(19) **Europäisches Patentamt**

**European Patent Office**

**Office européen des brevets**

(11) **EP 1 213 584 A1**

(12) **EUROPEAN PATENT APPLICATION**

(43) Date of publication:
**12.06.2002 Bulletin 2002/24**

(51) Int Cl.7: **G01N 33/12**

(21) Application number: **01113718.9**

(22) Date of filing: **05.06.2001**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **05.12.2000 GB 0029640**

(71) Applicant: **B.I.M. n.v.**
**8830 Hooglede-Gits (BE)**

(72) Inventors:
• **Pattyn, Martin**
**8800 Roeselare (BE)**
• **Descheerder, Filip**
**9800 Deinze (BE)**
• **Vanderhaeghen, Kristof**
**8501 Bissegem (BE)**

(74) Representative: **Bird, William Edward et al**
**Bird Goen & Co.,**
**Vilvoordsebaan 92**
**3020 Winksele (BE)**

(54) **Carcass segmentation apparatus and method by means of morphological operators**

(57)    A method and system are described for determining the quality of a carcass which inludes capturing an image of the carcass, identifying anatomical elements of the carcass in the image, examining each anatomical element in the image for an abnormality; and classifying each anatomical element in response to the examining step.

The system and method makes use of morphological operators to segment the image.

The results of the classification can be used to determine further processing of the carcass.

Fig. 14

EP 1 213 584 A1

# EP 1 213 584 A1

**Description**

FIELD OF THE INVENTION

The present invention relates to the automatic processing of articles, especially agricultural products such as animal carcasses and pieces of meat and especially the classification and/or sorting of such articles.

TECHNICAL BACKGROUND

Methods of classifying and/or articles such as agricultural products, for example meat or carcasses are known. EP 499 550 describes a method and apparatus for the classification of carcasses. Photographs are taken from various angles and are stored in the memory of a computer. These are used for classification purposes

US 5,793,879 describes a method of delineating the rib-eye muscle in beef in which morphological operators are used. However, the technique does not provide abnormality determinations for each anatomical element of the carcass.

SUMMARY OF THE INVENTION

The present invention may provide a method of determining the quality of a carcass, comprising the steps of:

capturing an image of the carcass;
identifying anatomical elements of the carcass in the image;
examining each anatomical element in the image for an abnormality; and
classifying each anatomical element in response to the examining step.

The identifying step may include the use of at least one morphological operator. The examining step of at least one anatomical element may include determining if the element is broken.

The present invention may also include a method of processing a carcass comprising the step of processing the carcass, e.g. segmenting the carcass, in accordance with the results of the classification. The segmented carcass may be used for different purposes, e.g. consumption whole, for making soup, etc.

The present invention may provide a system of determining the quality of a carcass, comprising:

an image capturing unit for capturing an image of the carcass;
image processor means for identifying anatomical elements of the carcass in the image and for examining each anatomical element in the image for an abnormality, and
decision means for classifying each anatomical element based on the output of the image processor means.

The present invention also includes a computer program product for carrying out the method steps of the invention when executed on a computer. The present invention also includes a computer readable data carrier including computer programming code segments for carrying out the methods of the present invention when executed on a computer.

The present invention will now be described with reference to the following drawings.

BRIEF DESCRIPTION OF THE DRAWINGS

Figs. 1A is a schematic side view of a production room in accordance with an embodiment of the present invention.
Fig. 1B is a schematic view of a production room of Fig. 1 looking towards the camera.
Fig. 1C is a schematic circuit diagram of the automatic processing system according to an embodiment of the present invention.
Fig. 2. is a representation of a chicken as captured by the camera of Figs. 1A to C.
Fig. 3 is a graph showing an analysis of the R signal of an RGB signal captured by the camera of Figs. 1A to C to determine the binary chicken image using a thresholding technique to distinguish parts of the image showing the chicken from parts showing any blood and the hook supporting the chicken.
Fig. 4 is a graph showing an analysis of a combined signal I derived from the RGB signal captured by the camera of Figs. 1A to C to determine the binary chicken image using a thresholding technique to distinguish parts of the image showing blood from those parts showing the hook supporting the chicken.

Fig. 5 is a representation of a binary chicken image.

Figs. 6 and 7 demonstrate the principles of erosion and dilation respectively.

Figs. 8 and 9 show the result of opening the binary chicken image with a knife shown in Fig. 8 for detection of the breast image.

Fig. 10 shows further processing of the breast image to remove the hole between the legs and at the neck of the binary chicken image.

Fig. 11 shows detection of the limbs of the binary chicken image.

Fig. 12 shows an analysis of the wing image to determine if it is broken using two linear decision functions.

Fig. 13 shows the detection of the wing tip by opening the wing image with a line.

Fig. 14 is a representation of the chicken image after it has been segmented into its various elements.

Fig. 15 shows an analysis of a combined signal Y of the RGB signal captured by the camera of Figs. 1A to C to detect blood spots on the wing using a decision function.

Fig. 16 is the result of the detection step of Fig. 15 showing the detection of a spot on the main part of the wing.

Fig. 17 is the result of detection of a spot on the legs.

## DESCRIPTION OF THE ILLUSTRATIVE EMBODIMENTS

The present invention will be described with reference to certain embodiments and drawings but the present invention is not limited thereto but only by the claims. The present invention will mainly be described with reference to the classification of chickens and chicken limbs such as wings and legs but the present invention is not limited thereto but may find advantageous use with many types of agricultural products, especially ducks, turkeys and other livestock.

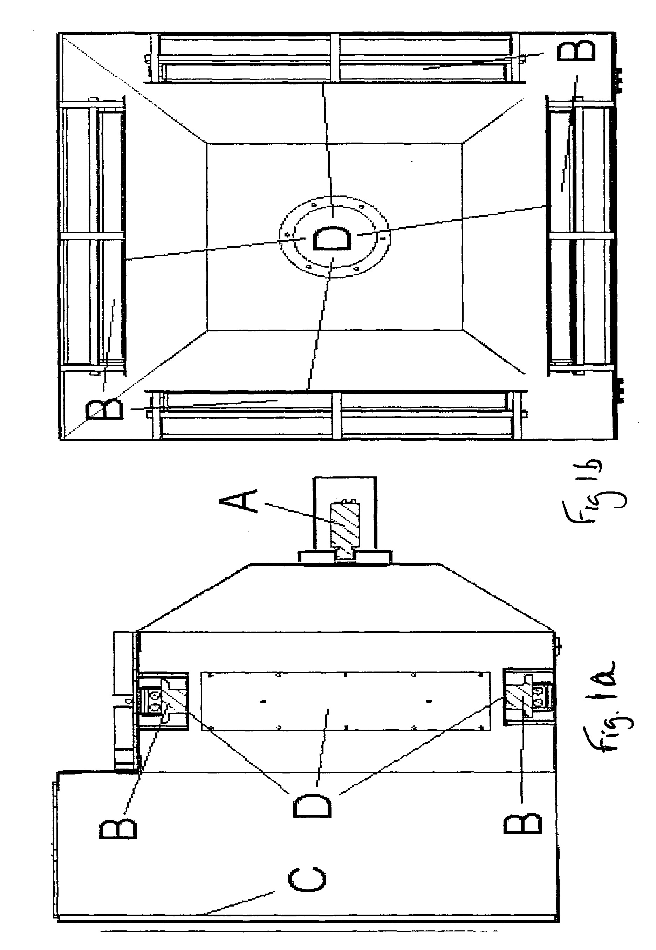

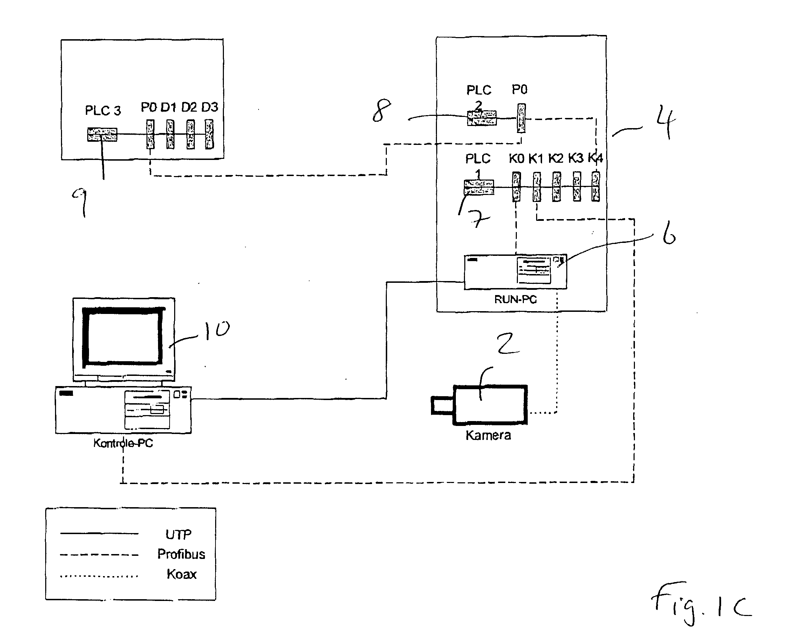

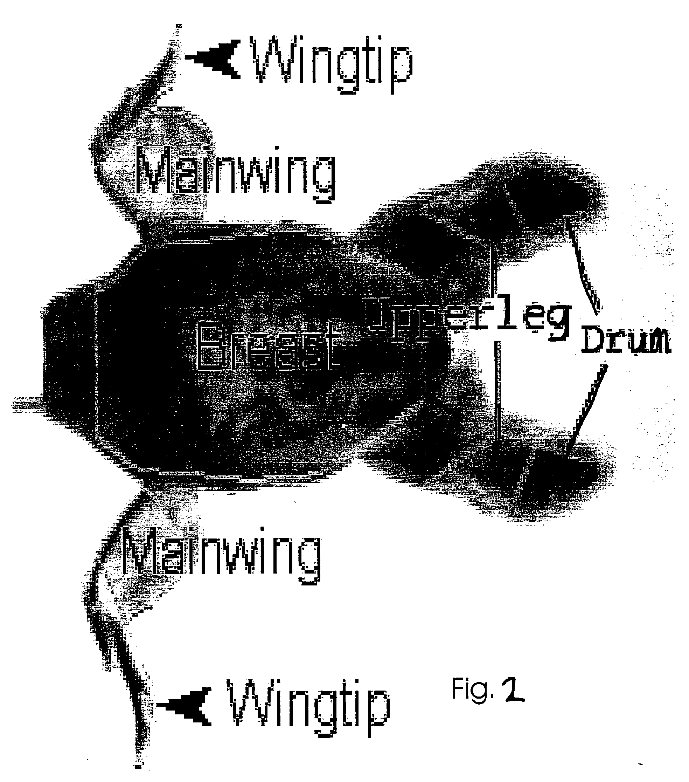

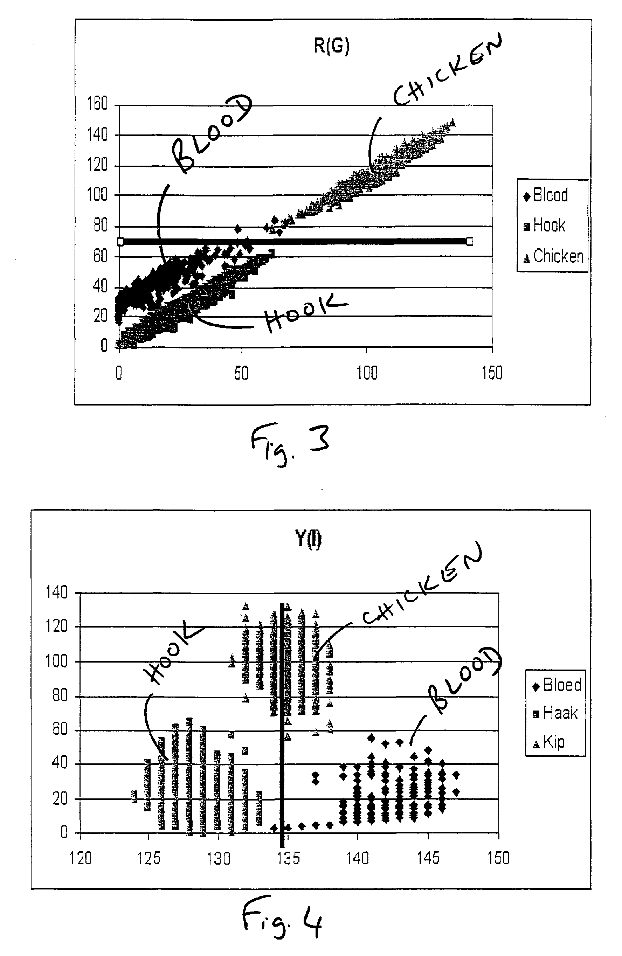

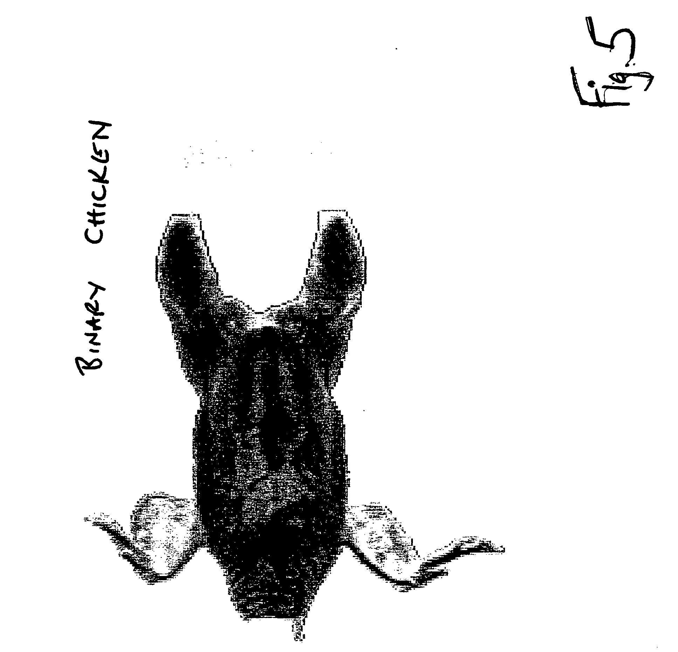

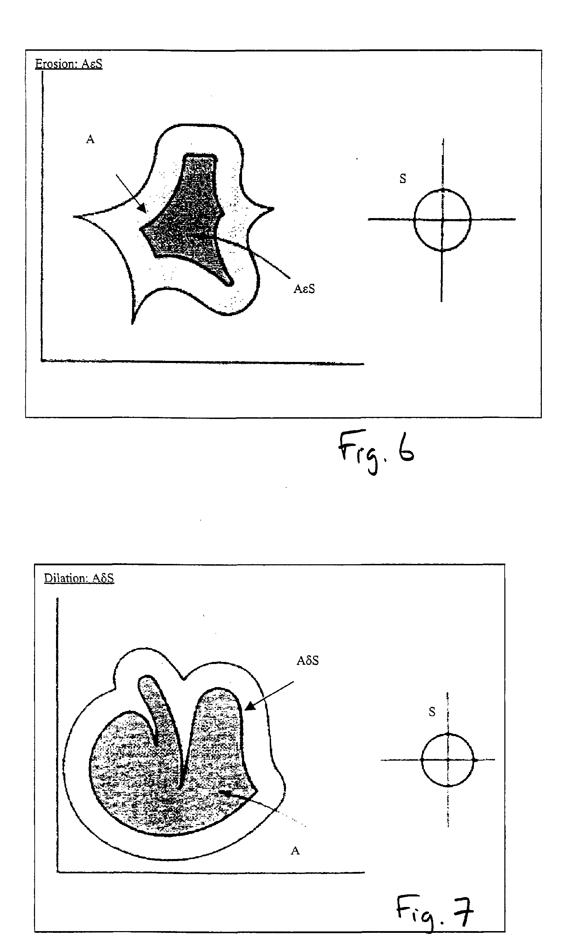

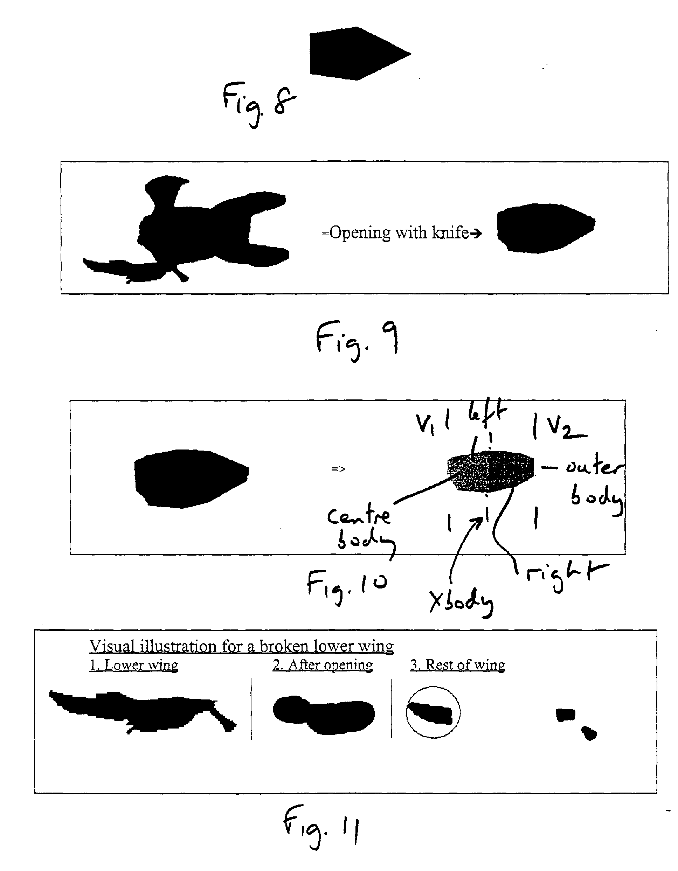

The present invention will be described with reference to the classification of plucked chickens being carried down a slaughter line, e.g. hung head down from one or more legs. In an embodiment of the present invention three processing rooms are provided: a technical room, a control room and a production room where the slaughter line is located. The production room is installed after the chicken chilling process. Along side the slaughter line and directed towards the slaughter line is located a camera ( see Fig. 1a, A) and one or more lamps (B). Generally, there will be several lamps arranged around the inside of the room as best shown in Fig. 1b in which 4 banks D of lamps, each of two lamps are shown. The lamps are preferably high frequency driven fluorescent lamps to a void flickering on the acquired image. The colour temperature of the lamps should be selected based on the colours of the articles to be photographed and the camera used. A colour temperature of 6500°K has been found to be suitable. More importantly, the frequency range of the light should ideally be as wide as the complete visual spectrum. The back C of the illumination box is preferably covered with or is made from a mat black material or paint. All materials should conform with food safety regulations. The other inner walls of the illumination box are preferably covered with or are made from a white material. Preferably, each lamp is covered by a diffuser, e.g. a mat white plastic plate D. This avoids direct illumination of the chickens and provides diffuse lighting without highlights.

Generally, the slaughter line will be controlled by an automatisation system shown schematically in Fig. Ic. The communication between the automatic classification system of the present invention and the automatisation system is preferably via a real-time communication protocol, for example Profibus™.

Generally, any type of high quality digital colour camera 2 may be used, e.g. a CCD camera. The camera 2 should preferably have asynchronous read-out which can be triggered by an external signal. The output is generally framed video data. The camera is preferably shuttered. The shutter time depends on the speed of the slaughtering line and is chosen so that each chicken image can be captured without delaying the line. The shutter time may be 1/250 s, for example when the slaughtering line is travelling at 63.5 cm/s. A signal amplitude with 256 gray-levels is preferred. The camera 2 preferably has an on-board LUT so that calibration correction can be carried out without any loss of processing power.

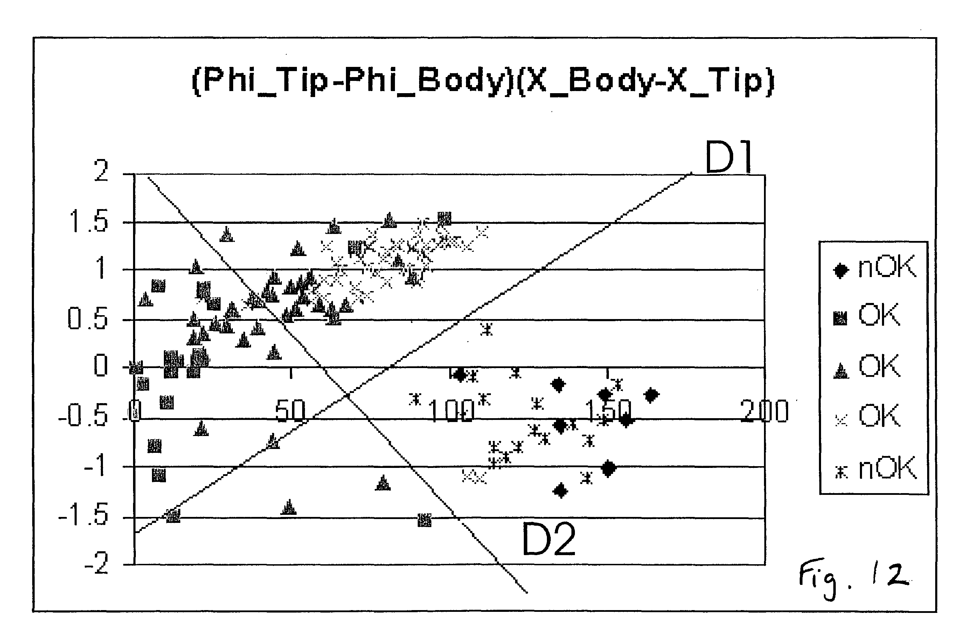

The slaughter line should be constructed so that each chicken is individually visible from the camera as shown in Fig. 2 (note in Fig. 2 the image has been inverted, black is white and vice versa to improve clarity and printing). The chickens should preferably be held so that they cannot rotate or swing thus preventing partial covering of a neighbouring chicken and improving image capture. Also the slaughtering line may be designed so that each chicken is held on a member which extends rigidly down from the slaughte line chain and the chain is made to dip into the illumination room so that an individual chicken is separated from its neighbours. A proximity sensor may be used to detect the arrival of a chicken and to synchronise the acquisition of the images at the same position each time.

The technical room is preferably close to the slaughter line. The computer system 6 used in a processing unit 4 to analyse the captured image and located in the technical room may be based on a 500 MHz Pentium III processor from Intel Corp; USA with a 100 MHz internal bus and 196 MB internal memory. The frame grabber in or attached to the processing unit 6 is preferably able to receive external hardware triggers and to support asynchronous grabbing in combination with the chosen camera 2. Several processing units 4 may be used. A PLC 7, 8, 9 may be used to divide incoming triggers from the slaughtering line over different processing units 4. A control room can be used for an operator to monitor the system and to adjust parameters if necessary. A monitoring application may be run on personal computer (PC) 10 running a suitable graphics interface such as Windows NT4, SP6, Microsoft Corp., USA. The operator can adjust the operation of the decision engine (see later) running on the PC 10. The PC 10 is preferably connected to the computer system 6 by means of a conventional UTP connection. The camera 2 is preferably connected to the computer system 6 via a coax cable. The Profibus™ connects the PLC system 7, 8, 9 to the computer system 6 and the PC 10.

The software on the PLC 7,8,9 includes the following functionalities:

- Send the trigger from the poximity sensor detecting the presence of a chicken ready for viewing to the processing unit 4, which is free at this moment.
- Send the output from the processing unit 4 at the appropriate time to the automation system of the plant.
- Send a copy of this output and additional information to the monitoring PC 10.
- Detect if all processing units 4 have correctly started up.
- Detect if a processing unit 4 has correctly stopped.
- Detect if a processing unit 4 is not responding. If this is the case, notify the automation system of the plant.
- Perform a hardware reset/start of one or more of the processing units 4, via a relay.

The Software on the monitoring unit 10 carries out the following tasks:
Learn mode that allows:

- The adjustments of the parameters of the decision engine.
- Initiate the command to upload the decision-parameters by a processing unit 4 via the UTP connection.
- Enables the user to monitor the results and logs them to a database.
- Enables the user to start and stop the processing units 4.

The software on the processing units 4 carries out the following tasks: A suitable computer pogramming language such as MSVisual C++, v.6 is used to program the functionalities, described below in the detailed description of the detection system. For standard image-processing components Halcon V was used. The Halcon operators were used in MSVisual C++, v.6, via the HalconCpp.h header and the HalconjCpp.lib library.

The steps in a method according to an embodiment of the present invention include:

1 - capture of a digital image of each chicken passing on a slaughter line;
2 - segmentation of the image into anatomical elements such as breast, main wing, wingtip, upper leg, drum;
3 - automatic inspection of each anatomical element to determine if any abnormalities are present, e.g. for the breast various spot types are considered to be abnormal, such as blood, manure, bruise, burn, pus spots. For wings and legs the absence or a break in the limb is considered an abnormality. That is, an abnormality is the presence of something which should not be there or the absence of something which should be there.
4 - determination of the quality of each element based on at least the presence or absence of an abnormality. This quality assessment can be a classification of each element such as a decision as how the element is to be processed. The classification may be a simple binary use/do not use decision or a more complex one. For example, a broken wing may be used for making chicken soup or for animal feed but would not be used whole for human consumption.

If a region does not show an abnormality it is classified "A". For an abnormality, e.g. spot type, a maximum major axis can be determined as well as a maximum surface area. If a particular spot has a major axis which is lower than a specified minimum the user may program the system to not take this spot into account. For all the spots of one region which do pass the minimum condition, the surface area of each spot is calculated. All the surface areas of all significant spots of one spot category of one region are added together and normalised, e.g. by dividing the sum by the quotient of the surface area of the anatomical element being considered and the maximum relative surface. The amounts obtained for the different types are added and if the result exceeds 1, the region is classified as inferior, e.g. type "B". Further, grades may be allocated depending on the total value. For the limbs, e.g. the wings, a missing wing may result in the complete chicken getting a "B" rating as it can no longer be used whole. A broken wing will be classified as "B" as will the complete chicken.

Information derived from the above analysis can include:

- the quality of each region
- the overall quality of the chicken
- if and how the chicken can be processed (e.g. cut into breast, wings, legs of which only some can be used whole for human consumption).

In addition the weight of the chicken may be determined and also recorded.

With respect to the image captured by the camera there is preferably compensation of illumination-variations. There are two reasons why this compensation is useful:

- Every illumination system has a variation of the intensity in function of time. This variation will affect the performance of the color-segmentation algorithms.
- When more than 1 processing unit is used, the units have to be calibrated one to another. This is necessary, because we are using a system with analog transfer of the images from the camera to the frame grabber. Tolerances in cables, input stages and A/D converters on the frame grabber cause these tolerances.

As shown in Fig. 2, the chicken may be divided into the following regions:

- Breast
- Main wing
- Wing-tip
- Upper leg
- Drum

It is a purpose of the present invention to provide a system which can automatically identify each one of these anatomical features of the carcass and to assess the quality of each anatomical feature. Once the image of a chicken has been captured the image is segmented into the various anatomical parts of the chicken: breast, wings, head, upper legs, drum in accordance with embodiments of the present invention.

The first step of the segmentation is done, based on colour information. This first step produces a binary image of the chicken, that is the binary chicken image has a single colour (e.g. black) compared to a uniform background (e.g. white). For the separation of the chicken-area from the background, two thresholds are preferably used:

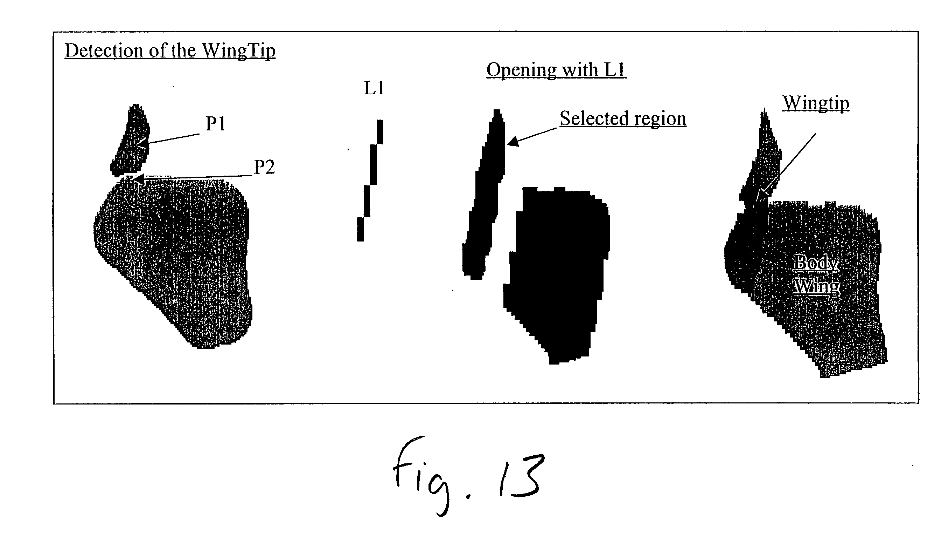

- One on the R-signal of the RGB signal captured by the camera : this threshold is used to separate the chicken from the hook supporting the chicken and the background (Fig. 3).
- One on the I- signal which is a combined signal using the RGB signals captured by the camera, where $1= 0.595*R - 0.276*G - 0.333*B$. The threshold on the I-signal is used to separate the blood from the hook (Fig. 4).

The result is shown in Fig 5.

Any spatial non-uniformity caused by the camera (CCD sensors + sensor read-out), lens and illumination is compensated. This non-uniformity varies in function of the position and the grey level. This means to have a perfect compensation, one would need a LUT for R, G and B, per pixel. Due to the amount of memory that this would take, and the complexity of the calibration process, the LUT for each pixel is approximated by a linear function but the present invention is not limited thereto. To measure variations in time and between different processing units 4, two gray references are placed in the illumination box. One with 20% gray, and one with 60% gray. The reference gray values are measured before the color segmentation algorithms are aligned.

For each pixel, the color I (R, G or B) is adjusted as follows:

$$I_{new} = a.I+b$$

For the gray references, the measured I-levels are $I_{20\%meas}$ and $I_{60\%meas}$, and the I-values measured at the moment of alignment are: $I_{20\%ref}$ and $I_{60\%ref}$. With this information, the following correction is executed for each pixel:

$$a.I+b, \text{ where } a=(I_{60\%ref}- I_{20\%ref})/ ( I_{60\%meas}- I_{20\%meas}) \text{ and } b=( I_{20\%ref}. I_{60\%meas} -$$

$$I_{60\%ref}. I_{20\%meas})/(I_{60\%meas} - I_{20\%meas})$$

This correction can be implemented in the LUT of the frame grabber. The advantage of this approach is that no processing time is lost. The grey values are monitored at every chicken, measured at the references. If the deviation between the measured value and the expected value is too high, the system will start the calibration procedure to update the LUT from the moment that the system is stopped.

Once this compensation is activated, the alignment of the color segmentation algorithm can be done and will perform stably in time and on different processing units 4. The reason this calibration is not combined with the spatial non-uniformity compensation, is that this calibration must be done before the detection of the binary chicken.

The second step of the segmentation separates the area, determined by the binary chicken image into a breast, two drums, two upper legs, two main wings and two wingtips. For this means morphogical operators like binary erosion, dilation, opening and closing are preferably used but the present invention is not limited thereto. Using mathematical morphological operators is a non-linear image analysis technique that concentrates on the geometric structure within an image. This technique can be used for image enhancement, segmentation, restoration, edge detection, texture analysis, particle analysis, curve filling and general thinning. General techniques of image analysis are given in the book "Traitement de l'image sur micro-ordinateur", Jean-Jacques Toumazet, Sybex, 1987.

*Erosion of discrete binary images*

A discrete binary image is a subset of a two-dimensional discrete binary space. With reference to Fig. 6, the erosion operator is characterized by a structured element (S). S is a discrete binary image with a reference point. The translation (+) of this structured element with a vector x (xx,yx) will map the reference point of S to the vector x. The subset, obtained by translation of a structured element S with a vector x is described by:

$$S+x=\{s+x-r, \text{ with } sES\},$$

where sES means that s is a member of S.

The erosion ($\varepsilon$) of a discrete binary image A with a structured element S can be described as:

$$A\varepsilon S =\{x, \text{ with } S+x \text{ is a subset of } A\}$$

*Dilation, opening and closing of discrete binary images*

The dilation ($\delta$) of a discrete binary image A with a structured element S can be described as:

$$A\delta S=\{x, \text{ where } S+x \text{ is a subset of } A\} \qquad (\text{see Fig. 7})$$

An opening is an erosion, followed by a dilation, using the same structured element. An opening can be used to separate or eliminate certain parts of a shape. A closing is a dilation, followed by an erosion. This can be used to attach different shapes, one to another, or to fill holes in a shape.

*The detection of the breast*

The body or breast of the chicken is detected by the use of an opening operator with a knife. This knife may have a shape as indicated in the Fig. 8. The orientation of this knife is determined by the average position of the chickens. The result can be a number of binary regions, of which the breast of the chicken is the largest (Fig. 9).

*Further processing of the breast*

Around the neck of the chicken and between the legs, there is a hole. This hole can have a colour (= an RGB-value) which can be the same as a colour (= part of the set of RGB-values) representative for certain types of spots ( which can be abnormalities). To avoid detecting these holes as spots, the spots at the left and right part of the breast are removed. This is done by drawing two vertical lines. One vertical line V1 is located at Xbody - Ra x 0,6 (to remove the hole at the position of the head), the other V2 is located at Xbody + Ra x 0.75 (to remove the hole between the legs), where Xbody is the x-coordinate of the center of gravity of the breast, and Ra is the 'Major axis' of the breast. Ra is defined as follows:

If the moments M20, M02 and M11 are normalized to the area, the Ra is calculated as:

$$Ra = \text{square root } (8.0 \text{ x } (M20+M02+\text{square root } ((M20-M02)^2 + 4.0 \text{ x } M11^2)))/2.0$$

where M20, M02 and M11 are calculated as follows:

X0 and Y0 are the coordinates of the center of the region under consideration.

Then the moments Mij are defined by:

$$Mij = SUM(\ (X0 - X)^i\ (Y0 - Y)^j),$$

wherein X and Y run through all pixels of the region under consideration.

The spots, which are not located within these two vertical lines, will not be taken into account.

Due to the complex shape of the breast, it is difficult to get uniform illumination over the whole breast. To solve this problem, the breast is divided into a center body, and an outer body. This is done by means of an erosion with an ellipse. The two axis of this ellipse depend pixel size of the average chicken. The center body itself is than once again divided into left and right center bodies. This is done by means of a vertical line, located at Xbody shown in Fig. 10.

*Detection of the limbs*

The intersection of the complement image of the breast with the binary chicken image results in the wings and the legs and possibly some regions left of the head being isolated. A triangle left of the head, of which the position and size is related to the center of gravity of the breast and Ra, and Rb of the breast, is removed. An opening on the result, with a circle, of which the radius depends on the pixel size of the average chicken, takes care of small particles, which are not part of the limbs. After this, the correct limb is identified (upper wing, lower wing, upper leg and lower leg). It is possible in this case that no wing, or only one wing is detected. This is because the wing(s) in question has been removed from the chicken, e.g. by a malfunction in the mechanics of the slaughter line.

*Separation of the wingtip to detect if the wing is broken (abnormality)*

The wingtip is in this case detected by:

-    An opening of the wing (1. Lower wing, Fig. 11) with a circle which gives the body of the wing (2. After opening, Fig. 11). The diameter of this circle depends on the average size of the chickens.
-    The intersection of the complement of the body of the wing with the wing itself, leaves the rest of the wing (3. Rest of wing, Fig. 11).
-    Of what is left, the most left object, which has a surface higher than a parameter (this parameter depends on the average size of the chickens) is selected. It is possible that no wing tip is left on one or both wings. If there is no wingtip left, the wing is considered not to be hanging completely open.

To detect if the wing is broken, the orientation of the tip, relatively to the body is considered. Also the distance between the gravity center of the tip and the gravity center of the breast is considered. These two parameters are used to determine if a wing is broken or not. Two linear decision-functions (D1 and D2), which are graphically illustrated in Fig. 12, are used to determine if the wing is broken or not.

If the wing is broken, the detection of the wingtip is not executed.

Detection of the wingtip (see Fig. 13).

This section is only used, if the wing is not broken

To detect the wingtip, an opening is performed with a line, L1 (Opening with L1, Fig. 13). To select the correct region after the opening, the region, which contains P1 is selected (Selected region, Fig. 13). After this, the complement of this wingtip is intersected with the wing. The region with the highest surface is selected (Body Wing, Fig. 13). Then again the intersection of the complement of this region with the wing is taken, and again the region which contains P1 is selected to provide the wing tip (Wingtip, Fig. 13).

To determine the line L1, two points P1 and P2 are required. First the gravity point of the wingtip (P1) is determined, which was used to determine if the wing was broken. Secondly a point (P2) is required, which is the gravity-point of the 'separation-zone' of the wingtip and the rest of the wing. This separation zone can be determined by the intersection of the tip and the rest of the wing, which have been dilated (morphological operator) with a circle (the radius of the circle is determined by the size of the average chicken).

The direction of the line used for the opening is determined by P1 and P2. The length of the line is four times the distance between P1 and P2.

*Further processing of the leg*

To take care of the non-uniformity of the illumination on the leg, the part where the illumination is uniform is selected. This is done by performing an erosion with a line, of which the orientation is along the major axis of the leg, and the size is related to the major axis of the leg, followed by a translation towards the angle in the same direction. Finally an erosion is done with a line. The orientation of this line is according to the minor axis. The size of the line is related to the minor axis.

*Separation of the leg into drum and upper leg*

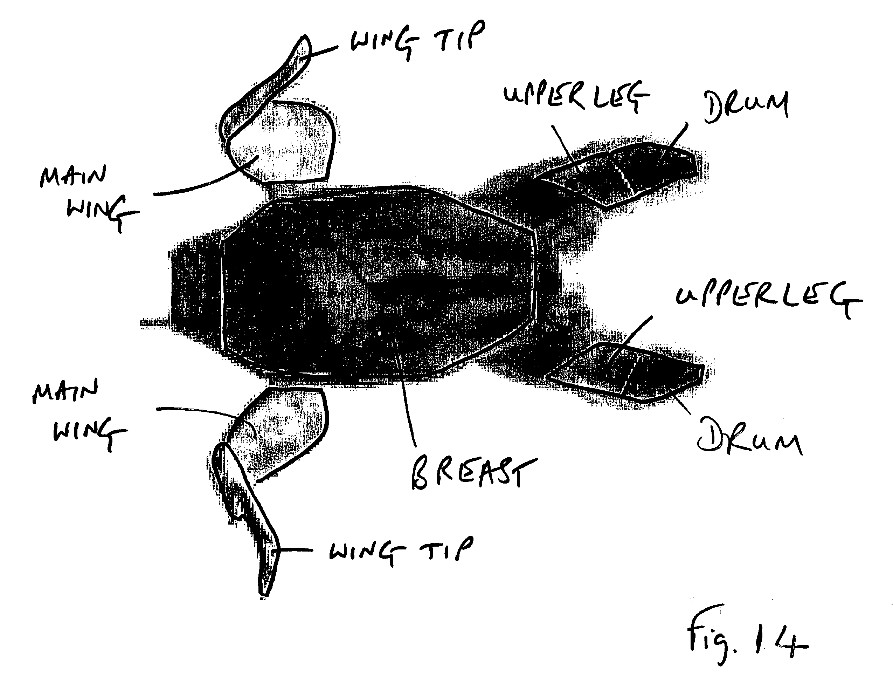

First the image of the leg is rotated, so that its major axis is in a horizontal position. Then a vertical line at the position of gravity point of the leg is used to separate the leg into the drum and the upper leg. And finally the result is rotated into back to the original orientation - see Fig. 14.

*Detection of the spots (colour abnomalities)*

Due to the different 3D-shape of the topological regions and the different types of spots to be detected on the various regions, different color-segmentation algorithms are used.

*Detection of spots on the wings*

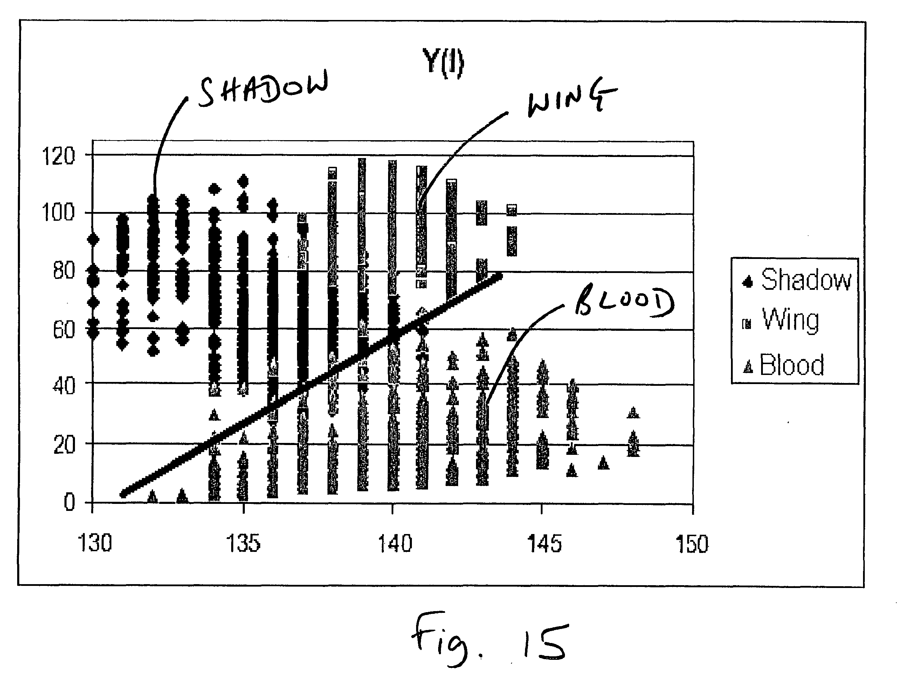

On the wings only 'blood' spots occur usually. The visual representation of the data illustrates that the color segmentation can be done by one linear decision functions in the Y-I space (Fig. 15).
The relation between Y, I and R, G and B is the following:

$$Y=0.299*R+0.587*G+0.144*B$$

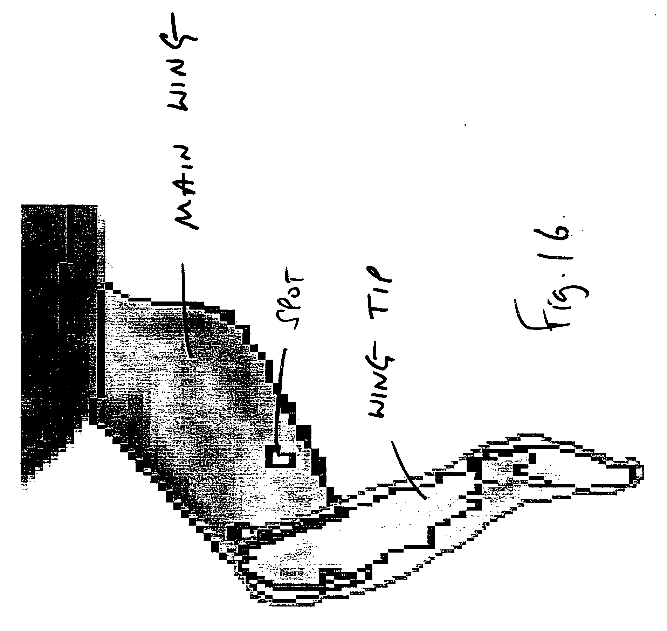

$$I = 0.595 * R - 0.276 * G - 0.333 * B \text{ (Fig. 16)}$$

*Detection of the spots on the legs*

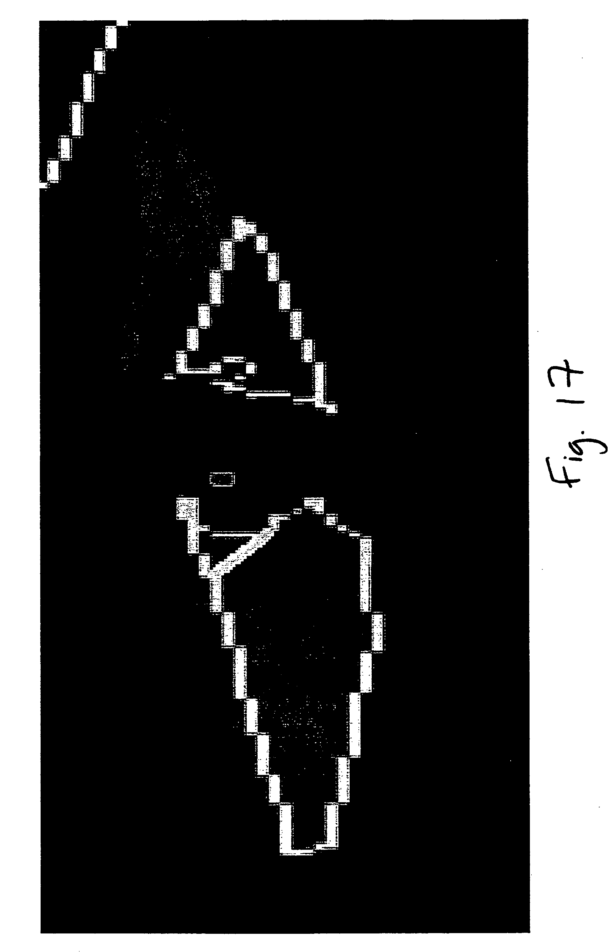

The principle is identical to that of the detection of spots on the wings. The parameters and the number of decision functions are different, as two types of spots are detected on the legs (Fig. 17).

*Detection of the spots on the body*

As there are a lot of different spot types on the body it is useful to use an LUT. A high-end color segmentation algorithm is preferred. Suitable color segmentation algorithms often use a very calculation-intensive algorithm. Such algorithms are not suited to be used on-line. A full color RGB LUT (255x255x255 bytes) is preferred.

*LUT construction*

Step 1: The algorithm starts from a set of RGB-samples, taken from different spot-types. To each RGB-sample a class is attached (this class corresponds to the spot-type).
Step 2: From the RGB-samples we construct a voting table, per class. The index to store or retrieve a value from this voting table is an RGB-value. At first, all votes (for each RGB-value) in the voting table are 0. Next the RGB-samples are considered one by one. Each time an RGB value occurs in the sample data, it's vote in the voting table is augmented by 1.
Step 3: To avoid that the 'strength' of a class is determined by the size of the class, or the number of samples taken for this class, a normalisation of the voting table is used. For this normalization, each vote is divided by the density of the class ($\rho$).
With:

$\rho$ = (total # of votes or samples) / ( volume of the class in RGB space).

The volume of the class in the RGB-space is estimated by:
square root (det ( Covariance matrix of the collection of RGB samples of the class under consideration ) ) ). This means that the class is considered to have an ellipsoïdal shape.

Step 4: From the different voting tables, we are able to determine a membership-value for each point of the RGB-space, for each different class. This membership is determined by:

- $Mik = Wk [\Sigma \, Ej / (\rho.(Dij+1))]$, if $(xi-xj)^2 + (yi-yj)^2 < (Dmax)^2$
- $Mik = 0$, if $(xi-xj)^2 + (yi-yj)^2 > (Dmax)^2$

the sum goes over all points j of class k.

With:

- Mik = membership of the point i to the class k.
- Ej = the number of votes in the point j.
- $Dij = sqrt((xi-xj)^2 + (yi-yj)^2)$.
- Wk = weight of class k.

To decide to which class label will be attached to a point i, the membership-values for the different classes are examined. The label of the class with the highest membership-value is attached to this point in the LUT.

The weight of class k (Wk) is normally set at 1 for each class. If we want a class k to grow, relatively to the other classes, this can be done by increasing Wk.

*Decision engine*

The inputs of the decision engine are the combined relative surfaces of the spots per type and per region. RSij = relative surface of the spot type i on the region j.
RSij = Sij / Sj, with Sij = sum of the surfaces of all spots of the type i on region j and Sj = the surface of region j.

Before this summation of regions is calculated, the spots with a 'major axis' lower than a threshold, are preferably omitted from the sum.

The definition of the major axis can be found above when the processing of the breast is described.

To take into account the importance of the different types of spots, a maximum relative surface of type i on region j $S_{ijmax}$ can be defined. To combine the information of the different types of spots into one parameter, the following decision-criterium is used for region j:

$$\text{If} \; \sum_{i=1}^{n} \; \frac{RS_{ij}}{S_{ijmax}} \; >=1 \text{ then region j is B-quality}$$

where n is the number of spot-types, occuring on region j.

In the case of the wings, this information is combined with the fact that the wing is broken or not.

The present invention may take the form a computer program product for carrying out any of the methods of the present invention when executed on a computer.

Furthermore, the present invention may take the form of a data carrier medium (e.g. a computer program product on a computer-readable storage medium) carrying computer-readable program code segments embodied in the medium. The code segments may carry out any of the methods of the present invention when executed on a suitable computer. The terms "carrier medium" and "computer-readable medium" as used herein refer to any medium that participates in providing instructions to a processor for execution. Such a medium may take many forms, including but not limited to, non-volatile media, volatile media, and transmission media. Non-volatile media include, for example, optical or magnetic disks, such as a CD-ROM or a storage device which is part of mass storage. Volatile media includes dynamic memory such as RAM. Transmission media include coaxial cables, copper wire and fiber optics, including the wires that comprise a bus within a computer, such as a bus within a computer system. Transmission media can also take the form of acoustic or light waves, such as those generated during radio wave and infra-red data communications.

Common forms of computer-readable media include, for example a floppy disk, a flexible disk, a hard disk, magnetic tape, or any other magnetic medium, a CD-ROM, any other optical medium, punch cards, paper tapes, any other physical medium with patterns of holes, a RAM, a PROM, an EPROM, a FLASH-EPROM, any other memory chip or cartridge, a carrier wave as described hereafter, or any other medium from which a computer can read.

These various forms of computer readable media may be involved in carrying one or more sequences of one or more instructions to a processor for execution. For example, the instructions may initially be carried on a magnetic disk of a remote computer. The remote computer can load the instructions into its dynamic memory and send the instructions over a telephone line using a modem. A modem local to the computer system can receive the data on the telephone line and use an infrared transmitter to convert the data to an infra-red signal. An infra-red detector coupled to a bus can receive the data carried in the infra-red signal and place the data on the bus. The bus carries data to main memory, from which a processor retrieves and executes the instructions. The instructions received by main memory may optionally be stored on a storage device either before or after execution by a processor. The instructions can also be transmitted via a carrier wave in a network, such as a LAN, a WAN, or the Internet. However, it is important that those skilled in the art will appreciate that the methods of the present invention are capable of being distributed as a program product in a variety of forms, and that the present invention applies equally regardless of the particular type of signal bearing media used to actually carry out the distribution. Examples of computer readable signal bearing media include: recordable type media such as floppy disks and CD ROMs and transmission type media such as digital and analogue communication links which may be used for downloading the computer program product.

**Claims**

1. A method of determining the quality of a carcass, comprising the steps of:

   capturing an image of the carcass;
   identifying anatomical elements of the carcass in the image;
   examining each anatomical element in the image for an abnormality; and classifying each anatomical element in response to the examining step.

2. The method according to claim 1, wherein the identifying step includes the use of at least one morphological operator.

3. The method according to claim 1 or 2 further comprising the step of segmenting the carcass image into anatomical elements.

4. The method according to any previous claim, wherein the examining step includes examining for any one of blood spots, a broken anatomical element, a missing anatomical element.

5. A method of processing a carcass comprising the step of segmenting the carcass in accordance with the results of any of claims 1 to 4.

6. A system of determining the quality of a carcass, comprising:

   an image capturing unit for capturing an image of the carcass;
   image processor means for identifying anatomical elements of the carcass in the image and for examining each anatomical element in the image for an abnormality, and decision means for classifying each anatomical element based on the output of the image processor means.

7. The system according to claim 6 wherein the image processor means includes means for using at least one morphological operator.

8. The system according to claim 6 or 7 wherein the image processor means comprises means for segmenting the carcass image into anatomical elements.

9. The system according to any of claims 6 to 8, wherein the image processing means comprises means for examining for any one of blood spots, a broken anatomical element, a missing anatomical element.

**10.** A data carrier medium carrying one or more computer readable code segments for controlling a processing device to carry out a method in accordance with any of the claims 1 to 4.

**11.** A computer program product for execution of any of the methods in accordance with any of the claims 1 to 4 on a computer system.

Fig. 1b

Fig. 1a

EP 1 213 584 A1

PLC 3    P0 D1 D2 D3

9

PLC   P0
2

8

4

PLC   K0 K1 K2 K3 K4
1

7

6

RUN-PC

10

Kontrole-PC

2

Kamera

| | |
|---|---|
| ——————— | UTP |
| — — — — — | Profibus |
| ·················· | Koax |

Fig. 1c

Fig. 2

R(G)

BLOOD

CHICKEN

HOOK

Legend: Blood, Hook, Chicken

Fig. 3

Y(I)

HOOK

CHICKEN

BLOOD

Legend: Bloed, Haak, Kip

Fig. 4

BINARY CHICKEN

Fig 5

Erosion: AεS

Frg. 6

Dilation: AδS

Fig. 7

*Fig. 8*

=Opening with knife→

*Fig. 9*

$V_1$ | *left* | $V_2$

=> — outer body

centre body

Xbody

right

*Fig. 10*

Visual illustration for a broken lower wing

1. Lower wing      2. After opening      3. Rest of wing

*Fig. 11*

(Phi_Tip-Phi_Body)(X_Body-X_Tip)

D1

D2

Fig. 12

nOK
OK
OK
OK
nOK

EP 1 213 584 A1

Fig. 13

EP 1 213 584 A1

UPPER LEG

DRUM

UPPER LEG

DRUM

UPPER LEG

WING TIP

BREAST

WING TIP

MAIN WING

MAIN WING

Fig. 14

Fig. 15

EP 1 213 584 A1

MAIN WING

SPOT

WING TIP

Fig. 16.

EP 1 213 584 A1

Fig. 17

**European Patent Office**

## EUROPEAN SEARCH REPORT

Application Number

EP 01 11 3718

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DE 196 19 099 C (HINZ AXEL ;EGER HORST (DE)) 13 November 1997 (1997-11-13) * the whole document * | 1-11 | G01N33/12 |
| X,D | US 5 793 879 A (BARRETT-LENNARD DAVID ET AL) 11 August 1998 (1998-08-11) * the whole document * | 1,2,5,7, 10,11 | |
| X | WO 95 21375 A (TULIP INT AS) 10 August 1995 (1995-08-10) * page 3, line 27 - page 4, line 22; figures * | 1,4-6,8, 9 | |

TECHNICAL FIELDS
SEARCHED (Int.Cl.7)

G01N
G06K

The present search report has been drawn up for all claims

| Place of search | Date of completion of the search | Examiner |
|---|---|---|
| THE HAGUE | 30 January 2002 | Savage, J |

EPO FORM 1503 03 82 (P04C01)

**ANNEX TO THE EUROPEAN SEARCH REPORT
ON EUROPEAN PATENT APPLICATION NO.**          EP 01 11 3718

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2002

| Patent document cited in search report | | Publication date | Patent family member(s) | | Publication date |
|---|---|---|---|---|---|
| DE 19619099 | C | 13-11-1997 | DE | 19619099 C1 | 13-11-1997 |
| US 5793879 | A | 11-08-1998 | AU | 665683 B2 | 11-01-1996 |
| | | | AU | 4036193 A | 18-11-1993 |
| | | | WO | 9321597 A1 | 28-10-1993 |
| | | | DE | 69329820 D1 | 08-02-2001 |
| | | | DE | 69329820 T2 | 07-06-2001 |
| | | | EP | 0636262 A1 | 01-02-1995 |
| | | | NZ | 251947 A | 26-11-1996 |
| | | | US | 6104827 A | 15-08-2000 |
| WO 9521375 | A | 10-08-1995 | WO | 9521375 A1 | 10-08-1995 |
| | | | EP | 0692090 A1 | 17-01-1996 |

EPO FORM P0459

For more details about this annex : see Official Journal of the European Patent Office, No. 12/82